# EUROPEAN PATENT APPLICATION

(11) **EP 1 767 847 A2**
(43) Date of publication of application: **28.03.2007**
(21) Application number: 06251472.4
(22) Date of filing: 20.03.2006
(51) Int. Cl.: F21S 13/12, F21V 35/00

(54) **Candle system comprising a container for storing items**

(30) Priority: 18.03.2005 US 663180 P; 11.07.2005 US 178862
(71) Applicant: For Your Ease Only, Inc., Chicago IL 60610 (US)
(72) Inventor: Greiner, Lori, Chicago, Illinois 60610 (US)
(74) Representative: Howe, Steven

(57) **Abstract**

A container for a candle, the container being configured as a candle cover or a candle base, wherein the container comprises at least one hollow cavity for storing items, e.g., matchsticks or fragrance oils.

## Description

### FIELD OF THE INVENTION

The present invention relates to a candle system, and more particularly, a candle system that includes a container for holding items such as matchsticks, matchbooks and other ignition sources, wax chips, incense and fragrance oils, as well as other items.

### BACKGROUND OF THE INVENTION

Candles are popular for home use, as well as in restaurants and other places where people wish to provide light, or simply a soothing and pleasant atmosphere. Candles can also be used as a means to deliver scents, such as air freshening smells or other agreeable odors. Many shapes and sizes of candles have been developed over the years.

For example, there are jar candles, votives and other similar candles where the candle burning element is formed within a solid container. These candles are designed to burn within the solid structure and, as the candle bums, the sides of the candles are not exposed. Also, the height of the structure (e.g., the jar) does not change as the candle bums. In some cases, the candle in the jar can be replaced with a newer candle after use. Jars commonly include a lid that covers the top of the jar and helps keep the candle dust free when not in use.

There are also stand-alone candles, such as pillars which are not formed within a solid containers. These candles often constitute entirely the burning element and are often placed inside of candle holders to maintain the candles in an upright formation and to catch hot wax that drips from the candle. Stand-alone candles differ from jar candles in that the height of the whole stand-alone candle varies with burn time.

Oftentimes, it is desirable to cover the tip of a candle to hide the appearance of a burnt wick, thereby maintaining the original appearance of the candle when the candle is not in use. Stand-alone candles often do not have a lid or any other type of cover and would especially benefit from a cover used for hiding a burnt wick. Jar candles sometimes have lids which are able to mask a burnt wick, but this is not always the case.

Candles are typically lit using matchsticks, matchbooks or other combustible materials. Generally, matchsticks are not sold along with a candle. A person lighting the candle often must separately purchase matchsticks, store them in a location remote from the candle and then retrieve matchsticks in order to light the candle. This can be a cumbersome and time-consuming process. Accordingly, there is a need for a candle configured to hold matchsticks or other ignition sources so a person can store matchsticks at the candle lighting site.

In addition, candles can be used to warm fragrance oils or melt wax chips or light incense. It would be desirable to provide a candle that can store these items so that they are easily accessible to a person desiring to use such things.

### SUMMARY OF THE INVENTION

According to a first aspect of the present invention, a container adapted to be coupled to a candle comprises at least one hollow cavity for storing items.

According to a second aspect of the present invention, there is provided a candle system including a candle and a candle container according to the first aspect of the invention.

According to a third aspect of the present invention, there is provided a decorative cover for a candle, arranged to be positioned about the top of a candle, the cover including at least one hollow cavity for storing an item.

According to a fourth aspect of the present invention, there is provided a candle system including a candle, a cover coupled to the top of the candle, and a base coupled to the bottom of the candle, the cover including a container for storing items.

In some embodiments, a container for a candle is provided. The container is coupled to a candle and includes at least one hollow cavity for storing items, e.g., ignition sources, oils, incense and melters such as wax chips. The container preferably includes at least one lid for covering the at least one hollow cavity. In many cases, the container also includes a striker located about an interior or exterior surface of the container. The container can be removeably or hingedly attached to the candle. The container can be a candle cover adapted to be positioned on a candle top or a candle base adapted to be positioned beneath a candle bottom. The container can also be positioned on a side of the candle or inside of the candle. The container is preferably decorative and in some cases is decorated as a design or a component of a design selected from the group consisting of a character, animal, plant, food item or scenery object. Alternatively, the container may not have an ornamental design but may be strictly utilitarian.

When the container serves as a candle cover, it is positioned about a top of a desired candle and includes at least one hollow cavity for storing an item. A bottom portion of the candle cover in some cases includes a groove having a diameter slightly larger than the diameter of a desired candle top so that the groove can be positioned about the candle top. Alternatively, the bottom portion of the candle cover may not include a groove but is instead flush with the candle top. The bottom portion of the candle cover can also be configured as a lid to be positioned upon a candle jar.

The cover is preferably decorative and is configured as a design or a component of a design selected from the group consisting of a character, animal, plant, food item or scenery object. In some cases, the cover is configured as a head of a character. The character head can also be configured as a lid to be positioned upon a candle jar or upon a lid of a candle jar. The cover can also be configured as a head of a character wearing a hat. In some cases, the character head includes the hollow cavity and the hat is configured as a lid for covering the hollow cavity of the character head. In other cases, the hat includes the hollow cavity and a lid is provided for covering the hollow cavity of the hat. In other cases, both the character head and hat each include a hollow cavity. For example, in such cases, the hat is configured as a lid for the character head and an additional lid is provided for covering the hat.

When the container serves as a candle base, a top portion of the candle base in some cases includes a groove having a diameter slightly larger than the diameter of a desired candle bottom so that the candle bottom can be received by the groove. Alternatively, the candle base may be flush with the candle bottom.

In some embodiments, a candle system is provided, which includes a candle and at least one container configured to be affixed to the candle, wherein the container comprises at least one hollow cavity for storing an item. The container is preferably decorative and is decorated as a design or a component of a design selected from the group consisting of a character, animal, plant, food item or scenery object. In some cases, the container is decorated as one component of a design and the candle is decorated as another component of the same design. For example, in certain cases, the container is a cover decorated as a character head and the candle is decorated as a character body or the container is a cover decorated as a character hat and the candle is decorated as a character head. In other cases, the container is a base decorated as character feet and the candle is decorated as a character body or the container is a base decorated as a character neck and the candle is decorated as a character head.

The candle system in some cases includes both a candle cover configured to be positioned about a candle top and a base configured to be positioned about a candle bottom. For example, in certain cases, the container comprises a head of a character wearing a hat, wherein either the head or the hat includes a hollow cavity for storing items and the base comprises a character feature beneath the head of the character, wherein the base includes a hollow cavity for storing items.

### BRIEF DESCRIPTION OF THE DRAWINGS

The present invention will be described, by way of example, with reference to the accompanying drawings, in which:
FIG. 1 depicts a candle jar according to an embodiment of the invention.
FIG. 2 depicts an assembled candle system according to an embodiment of the invention.
FIG. 3 depicts an exploded view of the candle system in FIG. 2.
FIG. 4 depicts an exploded view of a candle system according to another embodiment of the invention.
FIG. 5 depicts an exploded view of a candle system according to yet another embodiment of the invention.
FIG. 6 depicts an assembled candle jar according to another embodiment of the invention.
FIG. 7 is a top perspective view of the candle jar shown in FIG. 6 with its cover removed.
FIG. 8 is an exploded perspective view of a candle assembly according to another preferred embodiment of the invention.
FIG. 9 is a rear perspective view of a candle jar according to an embodiment of the invention.

### DETAILED DESCRIPTION OF THE PREFERRED EMBODIMENTS

A container for a candle is provided. The container is adapted for use with any type of candle, including a jar candle and a stand-alone candle and is positioned anywhere about a candle. In most cases, the container is positioned on top of the candle, beneath the candle or on a side of the candle. The container is attached or secured about the candle using any known attachment mechanism. In other cases, the container is positioned inside of a candle. For example; the container may be incorporated into a jar candle itself, such as a scoop located in the back of the jar. In cases where the container is positioned on top of the candle, a bottom portion of the container is preferably adapted to be positioned over a candle top, for example by including a groove having a diameter slightly larger than the diameter of a desired candle top so that the groove can be positioned about the candle top. Alternatively, the bottom portion of the container may be flush with the candle top so that it is merely stacked on top of the candle. In cases where the container is positioned beneath the candle, a top portion of the container is preferably configured to receive a candle bottom, for example by including a groove having a diameter slightly larger than the diameter of a desired candle bottom so that the candle bottom can be held upright within the groove. Alternatively, the top portion of the container may be flush with the bottom of the candle so that the candle is stacked on top of the container. In other cases, the candle bottom simply rests on top of the base. In cases where the container is positioned on the side of the candle, the container can have a side portion adapted to be fixed to the side of the candle.

The container can also serve other functions. For example, the container can be configured as a cover for covering a top of a candle to provide a decorative effect or to hide a burnt wick or the container can serve as a pedestal or base for supporting the candle. The container can also be decorative. For example, the container itself can be configured as a character or head of a character (e.g., an animal, bug or cartoon character), a plant (e.g., one or more flowers), a food item (e.g., a birthday cake or a fruit), a lighthouse, barn or other scenery object and the like. The candle can also be decorated according to a theme, such as Easter, Christmas, Halloween and the like. For example, in cases where the container is a character, it can be provided as a character sitting on top of the candle, sitting beneath the candle and holding the candle upright or even climbing and holding onto the side of the candle. In some cases, both the container and the candle are provided together to form a single decorative item. For example, the container can be positioned on top of a candle and serve as a character head and the candle can serve as a character body. The container can also be positioned beneath the candle, wherein the candle serves as a character head and the container serves as a character neck or the candle serves as a character body and the container serves as the character's base such as its feet. Any design or decoration is within the scope of the invention. While in many embodiments the container is decorative, the container is not required to be decorative and can have a strictly utilitarian purpose.

The container is also configured to hold items such as igniting material, e.g., matchsticks, or wax chips, fragrance oils or incense and the like. The container can hold or contain items in a variety of ways. In some cases, the container includes one or more hollow cavities for storing the material and one or more lids for covering the openings of the hollow cavities. Each lid can be entirely removed or opened by a pivotal connection to expose the opening of the hollow cavity. The container can also be provided with a flint strip or striker located on any desired surface. For example, the striker can be provided on an interior surface so that it is not in view while the container is affixed to the candle. Alternatively, the striker may be located on an exterior surface of the candle system.

The container can be fabricated of any suitable material such as ceramic, dolomite, resin, glass, porcelain, metal, plastic fabric, and woven materials. In some cases, the entire container is made of a single material whereas in other cases, the various components of the container can be made of one or more different materials. In addition, the container may have various shapes such as a mug or a building, for example.

In certain embodiments, the container is a cover for a candle. The cover may be configured to be positioned on the topmost portion of a desired candle. In some cases, the cover is completely detachable from the candle wherein in other cases, the cover is hinged to the candle top, e.g. the top of a candle jar. In either case, the cover includes a bottom portion that is configured to be positioned upon a candle. In some cases, e.g., for use with stand-alone candles, the bottom portion is configured to be mateable with the top of the candle. In other cases, e.g., for use with jar candles, the bottom portion is configured to be placed on top of the jar. For example, the bottom portion can be configured as a lid to be placed over an open jar or the bottom portion can be configured for being attached to a pre-existing lid of a jar.

In cases where the bottom portion of the cover is mateable with a candle, the bottom portion may include an interior groove that has a diameter or other dimensions for conforming to a desired candle top, the candle top being either the candle itself, the jar top of a jar candle or a lid of a jar candle. For example, in some cases, the interior groove has a diameter slightly larger than the diameter of the candle top so that the groove can be comfortably positioned about the candle top. One can position the candle cover upon a candle top at any time it is desired to provide decoration to or simply to hide a burnt wick of the candle.

The candle cover is preferably decorative in nature. For example, it can be configured as a decorative shape or design, thereby enhancing the appearance of the candle top. Any decorative shape or design is within the scope of the invention. In some embodiments, the candle cover is configured as a head of a character, e.g., a snowman. The character head can also be provided wearing a hat. In such cases, the character head is configured to store the ignition material and the hat is provided as a cover for the character head. For example, in some embodiments, the head includes a hollow cavity which stores materials such as matchsticks and the hat is configured as a lid which seals the opening of the cavity in the head. The hat serves as a lid, which can be either removed entirely from the opening or can be attached to the opening via a pivotal connection so that the opening may be exposed when the lid is ajar. When it is desirable to obtain a matchstick to light the candle, one lifts the hat off from the character head and retrieves a matchstick from inside the hollow cavity. The character head is also configured to be placed about a candle top in a manner already described.

In other embodiments, the candle cover is configured as a head of a character wearing a hat, wherein the hat is configured to store a material. The hat can be permanently attached to the character head so that both the head and hat are provided as a single piece or the hat can also be removable from the head. The hat in this embodiment includes a hollow cavity which stores the ignition material and a top lid can be provided to cover the hollow cavity of the hat. The top lid is preferably pivotally connected to the top of the hat so that an opening is exposed when the lid is pivoted ajar. When it is desirable to light the candle, one lifts the top lid and retrieves a match from the hollow cavity of the hat. The character head in this embodiment is also configured to be placed about a candle top.

In yet other embodiments, the candle cover is configured as a head of a character wearing a hat, wherein both the head and hat are each configured to store materials. In such cases, the head includes a hollow cavity which stores the material and the hat serves as a lid for the head. However, while the hat serves as a head, it also includes a hollow cavity which stores material and a top lid can be used to cover the hollow cavity of the hat. When lighting a candle, one can either remove the hat off from the character head to retrieve a matchstick from inside the character head or remove the top lid from the hat to retrieve a matchstick from inside the hat.

In additional embodiments, the container is a base or pedestal for a candle. The base is configured to be positioned on the bottommost portion of any desired candle. In some cases, the base is completely detachable from the candle wherein in other cases the base is hinged to the candle bottom, e.g., the bottom of a candle jar. In some cases, a top portion of the base is preferably configured to receive a candle bottom, for example by including a groove having a diameter slightly larger than the diameter of a desired candle bottom so that the candle bottom can be held upright within the groove. In other cases, the candle bottom, e.g. bottom of a candle jar, simply rests on top of the base. The base includes a hollow cavity which stores any type of material. An opening to the hollow cavity is provided anywhere about the base. In some cases, an opening to the hollow cavity is provided at the top of the base and the candle bottom can be positioned on top of the base over the opening to close it. In other cases, the opening is on the top of the base but a lid is provided for closing the opening, and the candle is adapted to be positioned in place on top of the lid. For example, the lid itself may include a groove for holding the candle bottom. In other cases, the lid may be flush with the bottom of the candle. In still yet other cases, the opening is provided on a side of the base, and a door for closing the opening is provided.

The base is preferably decorative in nature. For example, the base can be configured as any decorative shape or design, thereby enhancing the appearance of the candle bottom. Any decorative shape or design is within the scope of the invention. In some embodiments, the candle is configured as a character and the base depicts a bottom part of the character. For example, when the candle is configured as a character head, the base depicts a neck or body of the character. Likewise, when the candle is configured as a character body, the base depicts character feet. The base can be provided alone or along with other containers, such as a candle cover.

Embodiments of the invention also provide a candle system, which includes a candle and a container configured to be positioned about the candle. The container is configured to hold an ignition material according to any of the embodiments already described. The candle system can be provided as an assembly or kit, which includes the candle, container and/or ignition sources or other items for storing inside of the container. The candle system can be fabricated of any suitable material such as ceramic, dolomite, resin, glass, porcelain, metal, plastic fabric, woven materials, for example. In some cases, the entire candle system is made of a single material whereas in other cases, the various components of the candle system can be made of one or more different materials. In some cases, only the container is decorative whereas in other cases the entire candle system is decorative. For example, one or more candle containers can be configured as one or more components of a design wherein the candle can be configured as another component.

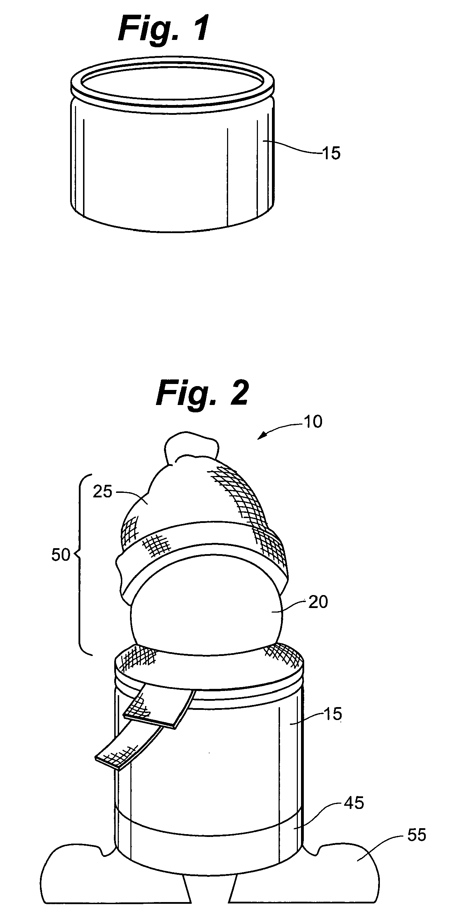

Preferred embodiments of the invention will now be described. In the illustrated embodiments, a candle system is provided for use with a standard jar candle 15 as shown in FIG. 1. The jar candle 15 may or may not include any kind of decorations about at least part of its circumference. The jar candle 15 may or may not include a lid. In the illustrated embodiment, a jar candle 15 without a lid is provided.

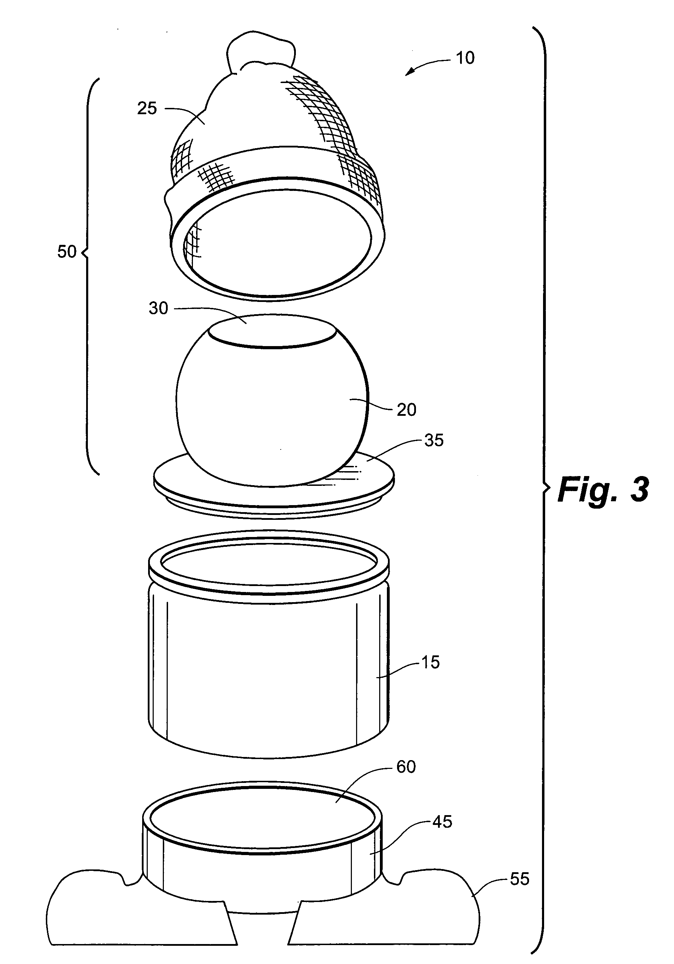

The candle system 10 according to the embodiments of the invention include ajar candle 15 and a container configured as a candle cover 50. FIG. 2 depicts the candle system in an assembled configuration wherein FIGS. 3-5 depict various embodiments of the candle system in an exploded configuration. Referring to FIG. 2, the candle cover 50 is decorated as a snowman head wearing a hat and is placed upon a jar candle 15, which in turn is placed upon a base 45 which is decorated as the character's feet 55. Referring to FIG. 3, the candle cover 50 shown in FIG. 2 includes a container 20 which has a top portion configured as a hollow cavity 30 and a bottom portion configured as a lid 35. The lid 35 may be provided with a rubber gasket to seal the lid 35 on the candle jar. The hollow cavity 30 can be used to store a variety of materials such as matchsticks or matchbooks or other ignition sources, wax chips, fragrance oils or incense. In addition, the hat 25 may be configured to have a depression within which wax chips, fragrance oils or incense may be contained. Of course an opening would need to be provided, preferably in the back of the character's head 20 to provide oxygen so that the candle can burn even with the cover 50 on the candle. This feature may be provided in all of the embodiments of the invention. The container 20 in this embodiment is configured as a snowman head. The lid 35 sits upon and may seal or close the interior space of the jar candle 15. A cover lid 25 is also provided and sits upon and closes the opening to the hollow cavity 30. The cover lid 25 in this embodiment is configured as a hat. The cover lid 25 may be omitted.

The base 45 in this embodiment is configured as feet of the snowman. Of course, the base 45 can be configured as any decorative or nondecorative design. Referring to FIG. 3, the container 45 includes a hollow cavity 60, which can also be used to store a variety of materials. The jar candle 15 sits upon the base 45 and may seal or close the opening to the cavity 60. In the illustrated embodiment, both a candle cover 50 and a base 45 are provided. Those skilled in the art will understand that both a candle cover and base can be provided or only one or the other need to be provided.

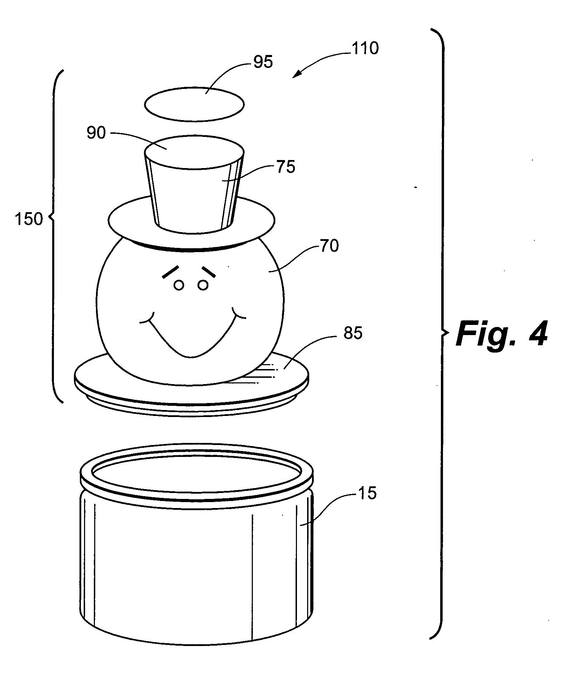

FIG. 4 depicts a candle system 110 including a jar candle 15 and a candle cover 150. The candle cover 150 in this embodiment is also decorated as a snowman head wearing a hat. The candle cover 150 includes a container 70 which has a cover lid 75 configured as a hat. In the embodiment of FIG. 4, the cover lid 75 is permanently attached or integral to the container 70 so that a snowman head wearing a hat is provided as a single piece. The cover lid 75 also serves as a container having a top portion configured as a hollow cavity 90 for storing materials such as matchsticks. A top lid 95 is also provided, which sits upon and closes the opening to the hollow cavity 90.

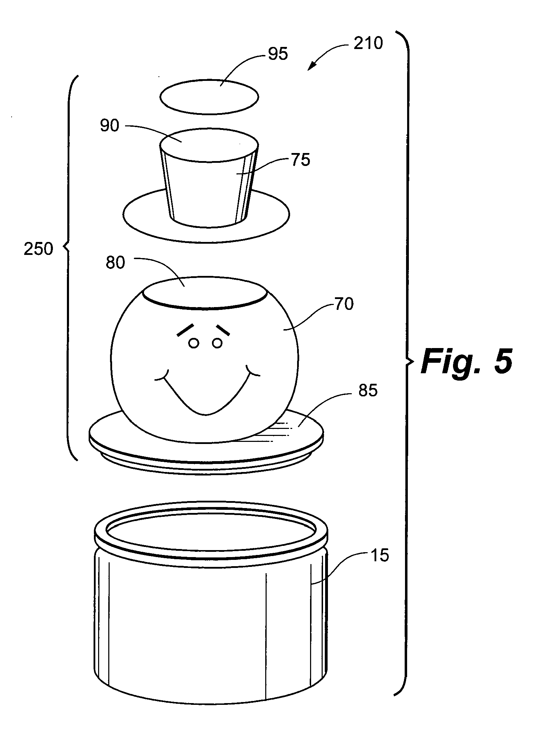

FIG. 5 depicts a candle system 210 having a candle cover 250 similar to the system of FIG. 4 wherein the cover lid 75 is releasably attached to the container 70. The container 70 in this embodiment has a top portion configured as a hollow cavity 80 for storing additional materials and the cover lid 75 sits upon and closes the opening to the hollow cavity 80. In the embodiment of FIG. 5, the candle system 110 contains two hollow cavities, 80 and 90 for storing material.

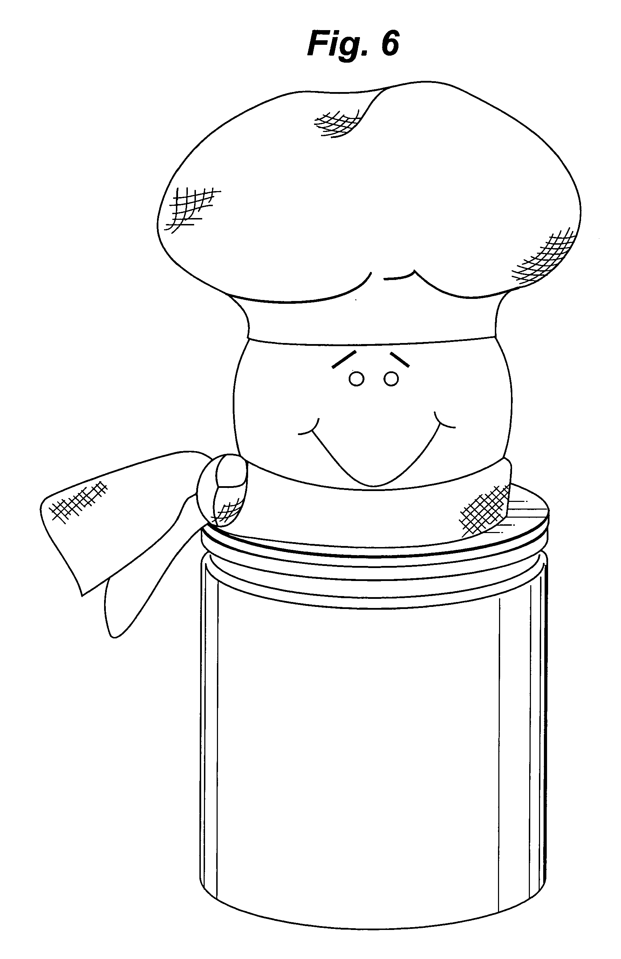

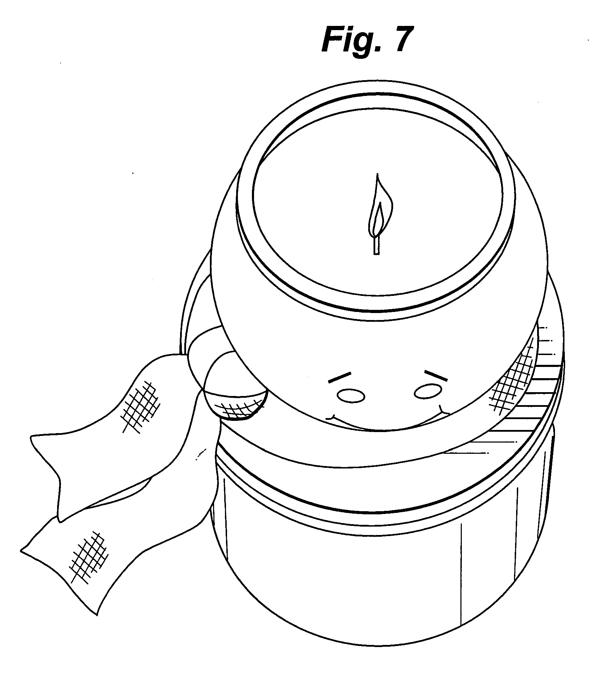

FIG. 6 depicts an assembled candle jar according to another embodiment of the invention. FIG. 7 is a top perspective view of the candle jar shown in FIG. 6 with its cover removed. In this embodiment, the candle jar represents a gingerbread man and the container is located under its hat.

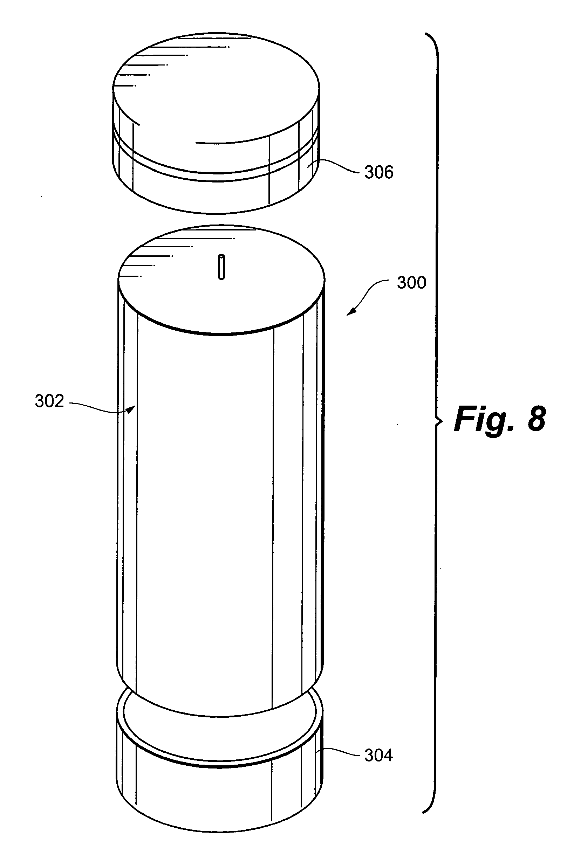

FIG. 8 is an exploded perspective view of a candle assembly according to another preferred embodiment of the invention.

The candle assembly 300 includes a pillar-type candle 302 and a base 304 and/or cover 306. The candle assembly 300 may be used with the base 304 or cover 306 alone with the pillar 302 or both the base 304 and cover 306 may be used simultaneously with the pillar 302. Either or both the base 304 and cover 306 may include a container therein.

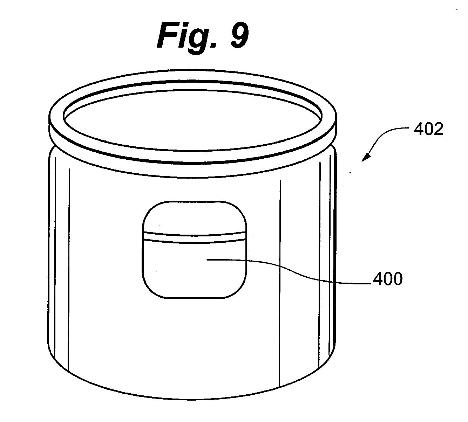

FIG. 9 is a rear perspective view of a candle jar according to an embodiment of the invention. The candle jar 400 includes a container 402 formed in the wall thereof that is in the form of a pocket in which items may be placed. In addition, a container such as that shown at 402 may be formed in any of the covers or bases previously described.

While preferred embodiments of the present invention have been described, it should be understood that various changes, adaptations and modifications may be made therein without departing from the scope of the appended claims.

## Claims

1. A container for a candle, the container arranged to be coupled to a candle and comprising at least one hollow cavity for storing items.

2. The container of claim 1 wherein the container is decorative.

3. The container of claim 2 wherein the container is decorated as a design or a component of a design selected from the group consisting of a character, animal, plant, food item or scenery object.

4. The container of any one of the preceding claims, wherein the container is removably coupled to the candle.

5. The container of any one of claims 1 to 3, wherein the container is hingedly coupled to the candle.

6. The container of any one of claims 1 to 3, wherein the container is a candle cover adapted to be positioned on a candle top.

7. The container of claim 6 wherein a bottom portion of the candle cover includes a groove having a diameter slightly larger than the diameter of a desired candle top so that the groove can be positioned about the candle top.

8. The container of claim 6 wherein a bottom portion of the candle cover is configured as a lid to be positioned upon a candle jar.

9. The container of any one of claims 1 to 3, wherein the container is a candle base adapted to be positioned beneath a candle bottom.

10. The container of claim 9 wherein a top portion of the candle base includes a groove having a diameter slightly larger than the diameter of a desired candle bottom so that the candle bottom can be received by the groove.

11. The container of any one of claims 1 to 3, wherein the container is adapted to be positioned on a side of the candle.

12. The container of any one of claims 1 to 3, wherein the container is positioned inside of the candle.

13. The container of any one of claims 1 to 3, further comprising at least one lid for covering the at least one hollow cavity.

14. The container of any one of the preceding claims, further comprising a striker located about an interior surface of the container.

15. The container of any one of the preceding claims, wherein the items are selected from the group consisting of ignition sources, oils, incense and wax chips.

16. The container of any one of the preceding claims, wherein the candle is contained in a jar.

17. A candle system, comprising:
a candle; and
at least one container according to any one of the preceding claims.

18. The candle system of claim 17 wherein the container is decorated as one component of a design and the candle is decorated as another component of the same design.

19. The candle system of claim 18 wherein the container is a cover decorated as a character head and the candle is decorated as a character body, or wherein the container is a cover decorated as a character hat and the candle is decorated as a character head, or wherein the container is a base decorated as character feet and the candle is decorated as a character body, or wherein the container is a base decorated as a character neck and the candle is decorated as a character head.

20. The candle system of claim 18 wherein the container comprises a candle cover configured to be positioned about a candle top and a base configured to be positioned about a candle bottom.

21. The candle system of claim 20 wherein the container comprises a head of a character wearing a hat, wherein either the head or the hat includes a hollow cavity for storing items and the base comprises a character feature beneath the head of the character, wherein the base includes a hollow cavity for storing items.

22. A candle system comprising:
a candle having a top and a bottom;
a cover coupled to the top of the candle; and
a base coupled to the bottom of the candle wherein the cover includes a container for storing items.

23. A cover for a candle, the cover being positioned about a top of a desired candle, wherein the cover is decorative and includes at least one hollow cavity for storing an item.

24. The cover of claim 23, wherein the cover is configured as a design or a component of a design selected from the group consisting of a character, animal, plant, food item or scenery object.

25. The cover of claim 24, wherein the cover is configured as a head of a character.

26. The cover of claim 25, wherein the head is configured as a lid to be positioned upon a candle jar or upon a lid of a candle jar.

27. The cover of claim 25 wherein the cover is configured as a head of a character wearing a hat.

28. The cover of claim 27 wherein the character head includes the hollow cavity and the hat is configured as a lid for covering the hollow cavity of the character head.

29. The cover of claim 27 wherein the hat includes the hollow cavity.

30. The cover of claim 29 further comprising a lid for covering the hollow cavity of the hat.

31. The cover of claim 27 wherein the both the character head and hat each include a hollow cavity.

32. The cover of claim 31 wherein the hat is configured as a lid for the character head and an additional lid is provided for covering the hat.
